(19)

Europäisches
Patentamt

European
Patent Office

Office européen
des brevets

(11) **EP 2 721 141 B1**

(12) **EUROPEAN PATENT SPECIFICATION**

(45) Date of publication and mention
of the grant of the patent:
**07.02.2018 Bulletin 2018/06**

(51) Int Cl.:
***C12M 3/00*** (2006.01)

(21) Application number: **12732900.1**

(86) International application number:
**PCT/US2012/042769**

(22) Date of filing: **15.06.2012**

(87) International publication number:
**WO 2012/174445 (20.12.2012 Gazette 2012/51)**

(54) **DEVICE AND METHOD FOR CULTURING CELLS IN A BIOMIMETIC ENVIRONMENT**

VORRICHTUNG UND VERFAHREN ZUR KULTIVIERUNG VON ZELLEN IN EINER
BIOMIMETISCHEN UMGEBUNG

DISPOSITIF ET PROCÉDÉ DE CULTURE DE CELLULES DANS UN ENVIRONNEMENT
BIOMIMÉTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.06.2011 US 201161497376 P**

(43) Date of publication of application:
**23.04.2014 Bulletin 2014/17**

(73) Proprietor: **The Charles Stark Draper Laboratory,
Inc.
Cambridge, MA 02139 (US)**

(72) Inventors:
  • **CHAREST, Joseph L.
Boston, Massachusetts 02130 (US)**
  • **FROHLICH, Else
Brookline, Massachusetts 02446 (US)**
  • **BORENSTEIN, Jeffrey T.
Newton, Massachusetts 02464 (US)**

(74) Representative: **FRKelly
27 Clyde Road
Dublin D04 F838 (IE)**

(56) References cited:
  **WO-A1-95/24464          WO-A1-97/05238
  WO-A2-2006/037033     WO-A2-2010/124227
  GB-A- 2 262 538          US-A1- 2001 003 653**

  • **CLARK P ET AL: "TOPOGRAPHICAL CONTROL
    OF CELL BEHAVIOUR: II. MULTIPLE GROOVED
    SUBSTRATA", DEVELOPMENT, COMPANY OF
    BIOLOGISTS, CAMBRIDGE, GB, vol. 108, 1
    January 1990 (1990-01-01), pages 635-644,
    XP002944530, ISSN: 0950-1991**
  • **CLARK P ET AL: "TOPOGRAPHICAL CONTROL
    OF CELL BEHAVIOR I. SIMPLE STEP CUES",
    DEVELOPMENT, COMPANY OF BIOLOGISTS,
    CAMBRIDGE, GB, vol. 99, no. 3, 1 March 1987
    (1987-03-01), pages 439-448, XP009162069, ISSN:
    0950-1991**

EP 2 721 141 B1

## Description

## FIELD OF THE INVENTION

In general, the disclosure relates to a an apparatus comprising a microfluidic flow channel for culturing cells in a biomimetic environment and a method for simulating organ performance using the apparatus.

## BACKGROUND OF THE INVENTION

Kidney cells grown under controlled *in vitro* conditions have a wide range of potential applications in the study of kidney functionality, the production of medical devices, and the testing of pharmaceuticals. Kidney cell cultures allow biologists to study the functions of kidney-related cells and observe cells' responses to various conditions. Highly controlled nephritic environments can be used to perform some functions of the kidney to assist a patient with renal disease, and could be used by tissue engineers to generate specific kidney tissues for implantation into a patient with renal disease. Furthermore, kidney cell cultures can be used in the development of pharmaceuticals for kidney therapy and for testing kidney toxicity of pharmaceuticals. A controlled *in vitro* environment can be used for other types of cells as well, such as for eliciting desired cell functions of stem cells.

Each of these applications is benefitted by conditions that cause the *in vitro* cells to accurately replicate cells *in vivo.* While devices and methods exist for culturing kidney cells, traditional *in vitro* kidney cell environments are static, failing to account for shear stress experienced in nephrons. Furthermore, previous *in vitro* environments for kidney cell cultures do not provide biomimetic cues such as those provided by the extracellular matrix (ECM), so cells grown in the previous environments do not have the phenotype and morphology, for example cell shape or arrangement, they have *in vivo.* Proper arrangement of kidney cells in nephrons, particularly the formation of cell-to-cell junctions between cells, is necessary for kidney cells to perform their filtering and absorption functions. Thus, kidney cells grown in previous apparatuses fail to mimic the conditions of a nephron. WO2006/037033 describes a multi-compartment microfluidic device for enabling fluidic isolation among interconnected compartments and accomplishing centrifugal positioning and / or patterned substrate positioning of biological specimens within the device.

Clark P et al. studied topographical control of cell behaviour on multiple grooved substrata. WO2010/124227 describes a micro fluidic device having a cell capture surface and a flow modification surface under conditions that allow a CTC to bind.

## SUMMARY

There is therefore a need for a biomimetic device that can be used to grow a kidney cell culture that better replicates *in vivo* conditions of a nephron. In particular, an apparatus that controls the geometry and arrangement of cells in a flow channel can be used to create an *in vitro* environment that more closely mimics *in vivo* conditions than previous apparatuses. Microfabrication and micromolding techniques can be used to produce artificial cellular substrates with micro-, sub-micro-, and nano-topographical patterns that mimic the effect the extracellular matrix (ECM) has upon kidney cells. The design of the topographical surface allows close control of cells grown atop the substrate. This surface patterning, along with additional flow channel parameters such as channel height, channel cross-sectional area, and flow rate, can be used to create highly controlled *in vitro* conditions that closely mimic the *in vivo* environment of specific cell types.

Accordingly, the invention provides an apparatus comprising:

a microfluidic flow channel having at least one surface with ridges and grooves parallel to the direction of flow through the flow channel and having-two different topographical patterns formed therein, the two different topographical patterns being distinct regions having different groove widths, the two topographical patterns of the surface are of micron-scale, the two different topographical patterns used to mimic an in vivo structure having two distinct cell types or arrangements; and

an inlet in fluid connection with the flow channel for allowing fluid to flow into the flow channel.

Typically the two different topographical patterns of the surface are selected to promote increased adhesion of cells in the cell layer to the at least one surface.

Typically the apparatus further comprises a fluid source for flowing a fluid through the flow channel via the inlet, wherein the fluid induces a shear stress upon the cell layer.

The flow channel can be disassembled for examining the cell layer after flowing the fluid through the flow channel; or wherein the fluid source is configured to flow the fluid at a flow rate that results in a level of shear stress on the cell layer that is less than $10^{-6}N/cm^2$ (0.1 $dyn/cm^2$).

The two different topographical patterns of the surface comprise ridges having a width of about 5 micron or less.

The two different topographical patterns of the surface comprise grooves having a widths in the range of 20 nm to 5 microns.

Typically the apparatus comprises a cytophilic substance disposed on a portion of a substrate for growing the cell layer in the portion of the substrate, and wherein the portion of the substrate forms a surface of the flow channel.

Alternatively or additionally the apparatus comprises a cytophobic substance disposed on a portion of a surface of a substrate for preventing growth of the cell layer in the portion of the substrate, and wherein the portion of the substrate does, or does not, form a surface of any flow channel.

The two different topographical patterns of the surface may cause the arrangement, behavior, or morphology of the cell layer to replicate an arrangement, behavior, or morphology of cells in a kidney.

Typically the at least one surface of the second flow channel has a topography formed therein.

The invention also provides a method for simulating organ performance comprising:

providing a flow apparatus comprising:

a microfluidic flow channel having at least one surface with ridges and grooves parallel to a direction of the flow channel and having two

different topographical patterns formed therein, the two different topographical patterns being distinct regions having different groove widths, , the two topographical patterns of the surface are of micron-scale, the two different topographical patterns used to mimic an *in vivo* structure having two distinct cell types or arrangements; and

a cell layer, having the two distinct cell types or arrangements over the two different topographical patterns

selecting a flow rate for a fluid that, when flowed through the flow channel,
results in a level of shear stress on the cell layer that causes cells in the cell layer to mimic the morphology, behavior, or arrangement they would exhibit *in vivo*; and
introducing a fluid flow through the flow channel at the selected flow rate. Preferably the flow channel is shaped such that the fluid flow results in a plurality of shear stress levels along a length of the flow channel, the method further comprising:
selecting a flow rate that results in a plurality of levels of shear stress on the cell layer that mimics shear stress levels that the cells in the cell layer would experience in different positions of the organ *in vivo*.

Typically the fluid contains a therapeutic agent, and the method further comprising analyzing the cells to determine the efficacy of the therapeutic agent on the cell layer.

The biomimetic flow apparatus of the invention is defined according to independent claim 1 and includes a microfluidic flow channel and an inlet in fluid connection with the flow channel for allowing fluid to flow into the flow channel. The flow channel has at least one surface with two different topographical patterns formed therein.

The topographies of the at least one surface of the flow channel are selected to cause the cells in a cell layer disposed above the surface to achieve an arrangement, behavior or morphology. The cell arrangement, behavior or morphology is determined at least in part by the topography of the at least one surface.

The topographies of the surface are selected to promote increased adhesion of cells in the cell layer to the at least one surface. The topographies of the surface may include ridges having a width of about 5 μm or less. At least one surface of the flow channel has at least two different topographies. The surface topographies can cause the arrangement, behavior or morphology of the cell layer to replicate an arrangement, behavior or morphology of cells in a kidney.

In some embodiments, the apparatus also includes a fluid source for flowing a fluid through the flow channel via the inlet. The fluid induces a shear stress upon the cell layer. The shear stress on the cell layer may result in a level of shear stress that mimics a shear stress the cell type experiences *in vivo.* In some other embodiments, the shear stress on the cell layer may be less than $2^{-6}$N/cm$^2$ (0.02 dyn/cm$^2$). The channel may be shaped such that flowing the fluid through the flow channel results in a plurality of shear stress values along a length of the flow channel. After the fluid is flowed, the flow channel can be disassembled for examining the cell layer.

In some embodiments, a cytophilic substance is disposed on a portion of a substrate for growing the cell layer in that portion, which forms a surface of the flow channel. In some embodiments, a cytophobic substance is disposed on a portion of a surface of a substrate for preventing growth of the cell layer in the portion of the substrate; this portion of the substrate may or may not form part of a surface of the flow channel.

In some embodiments, the apparatus includes at least a second flow channel having at least one surface with a topography formed therein.

According to another aspect, the invention relates to a method for simulating organ performance according to independent claim 11. The method includes the steps of providing a flow apparatus having a surface with two different topographical patterns and a cell layer with a two distinct arrangement, behavior, or morphology. The method also includes the steps of selecting a flow rate that results in a plurality of levels of shear stress on the cell layer that causes cells to mimic the morphology, behavior, or arrangement they would exhibit *in vivo* and introducing a fluid flow through the flow channel at the selected flow rate. The flow apparatus may be similar to the flow apparatus described above.

The method may include disassembling the flow channel and examining the cell layer after flowing the fluid through the flow channel. The cells may be fixed to preserve the cell layer for analysis. If the fluid flowed through the flow channel contains a therapeutic agent, the method further includes analyzing the cells to determine the efficacy of the therapeutic agent on the cell layer. If the fluid contains a potential toxin, the method further includes analyzing the cells to determine the toxicity of the potential toxin to the cell layer.

Also described is a system for creating biomimetic flow. The system includes a flow channel having at least one surface with two different topographical patterns formed therein. The topographies of the surface of the flow channel are selected to cause cells in a cell layer grown on the surface to achieve an arrangement, behavior, or morphology and the topographies may promote increased adhesion of cells in the cell layer to the at least one surface. The system also includes an inlet and a fluid injection system. The inlet is in fluid connection with the flow channel for allowing fluid to flow into the flow channel. The fluid injection system causes fluid to flow through the flow channel to induce a shear stress upon the cell layer.

The system includes a temperature controller for maintaining the temperature of the microfluidic flow channel at a nearly constant temperature. In some embodiments, the system includes observational equipment for observing cells in the cell layer. If the flow channel is formed from at least a first flow channel layer and a second flow channel layer, and the system may also include a mounting apparatus for sealing the first flow channel layer to the second flow channel layer.

## BRIEF DESCRIPTION OF THE DRAWINGS

The apparatus and method may be better understood from the following illustrative description with reference to the following drawings in which:

Figure 1A is a diagram of a kidney cell culture grown on a flat surface;

Figure 1B is a diagram of a kidney cell culture grown on a substrate with topographical patterning;

Figure 1C is a picture of a kidney cell culture grown on a flat surface;

Figure 1D is a picture of a kidney cell culture grown on a substrate with topographical patterning;

Figure 2A is an exploded solid model of an apparatus with biomimetic flow channels,;

Figure 2B is a solid model of the assembled apparatus with biomimetic flow channels from Figure 2A,;

Figure 2C is a block diagram of a flow system for use with the biomimetic flow apparatus of Figure 2B,;

Figure 3 is a flowchart for a method for creating and using one of the biomimetic flow channels of Figure 2;

Figure 4 is a series of diagrams illustrating a method for producing a substrate with topographical patterning;

Figure 5 is a flowchart for a method for creating cytophilic and cytophobic regions on a surface and growing cells on the surface.

Figures 6A, 6B, 6C, and 6D are perspective views of cross sections of biomimetic flow channels with different topographical characteristics;

Figure 7A is a perspective view of a substrate having a gradient in its topographical pattern;

Figures 7B, 7C, and 7D are top views of three illustrative embodiments of flow channel surfaces having variations in their topographical patterns along the channel;

Figures 8A, 8B, 8C, 8D, 8E, and 8F are perspective views of illustrative embodiments of six substrates having different topographical patterns;

Figures 9A and 9B are flowcharts for methods for using one of the biomimetic flow channels of Figure 2;

Figure 10A, 10B, 10C and 10D are schematics of a biomimetic flow apparatus of the invention;

Figure 10E is a phase contrast image of cells within the channel adhered to the ECM-coating region.

Figure 11A is photograph of a nickel alloy mold used to fabricate a ridge/groove pattern surface;

Figure 11B is a photograph of a polystyrene substrate;

Figure 11C is a photograph of the edge profile of the polystyrene substrate shown in Figure 11B;

Figure 12A shows fluorescently-labeled nuclei of confluent layers of HK-2 cells grown on either blank (top row) or topographical (bottom row) substrates exposed to 2 hours of either 0, 0.02 or 1.0 $dyn/cm^2$ FSS. The arrow indicates the direction of grooves on the topographical substrate;

Figure 12B is a bar graph showing the % cells aligned to grooves. The first three bars represent cells grown on blank substrate (no topography) and exposed to 0, 0.02 or 1 $dyn/cm^2$ FSS, respectively. The second three bars represent cells grown on topographical substrate and exposed to 0, 0.02 or 1 dyn/cm² FSS, respectively;

Figure 13A and 13B show representative images of ZO-1 expression for cells cultured on blank or topographical substrates and exposed to either 0, 0.02 or 1 dyn/cm² FSS;

Figure 14A is a bar graph showing ZO-1 intensity integrated along cell perimeters and normalized by cell perimeter. The first three bars represent cells grown on blank substrate (no topography) and exposed to 0, 0.02 or 1 dyn/cm² FSS, respectively. The second three bars represent cells grown on topographical substrate and exposed to 0, 0.02 or 1 dyn/cm² FSS, respectively;

Figure 14B is a bar graph showing standard deviation of ZO-1 intensity measured along cell perimeters. The first three bars represent cells grown on blank substrate (no topography) and exposed to 0, 0.02 or 1 dyn/cm² FSS, respectively. The second three bars represent cells grown on topographical substrate and exposed to 0, 0.02 or 1 dyn/cm² FSS, respectively.

## DESCRIPTION OF CERTAIN ILLUSTRATIVE EMBODIMENTS

To provide an overall understanding of the invention, certain illustrative aspects of the disclosure will now be described, including apparatuses and methods for culturing cells in a biomimetic environment.

A biomimetic flow apparatus includes a flow channel having a fluid inlet and a fluid outlet. To create a flow channel that replicates an organ structure, such as a nephron, a culture of the organ's cells is grown on at least one side of the flow channel. In the body, the extracellular matrix (ECM) structurally supports the cells of a nephron and causes the cells lining a nephron to align with each other in a particular arrangement. In a biomimetic flow channel, the proper choice of topography for the surface upon which the cells are grown causes the cells to have the alignment, morphology, behavior and/or arrangement that would be created by the ECM. A biomimetic flow channel with a topography can be used to replicate not only kidney structures, but also other structures that experience flow. For example, the use of a biomimetic flow apparatus may prove useful for the study of such cells as: the urothelial cells in the bladder; brush border cells and intestinal epithelium in the small and large intestinal; vascular endothelial cells in the heart, veins, arteries, and capillaries; endothelial cells in the capillaries and ducts of the lymphatic system; hepatic epithelial cells or any other structure which experience fluid flow. When studying many of these systems it could be beneficial for *in vitro* cells to better mimic the morphology, alignment or arrangement of *in vivo* cells.

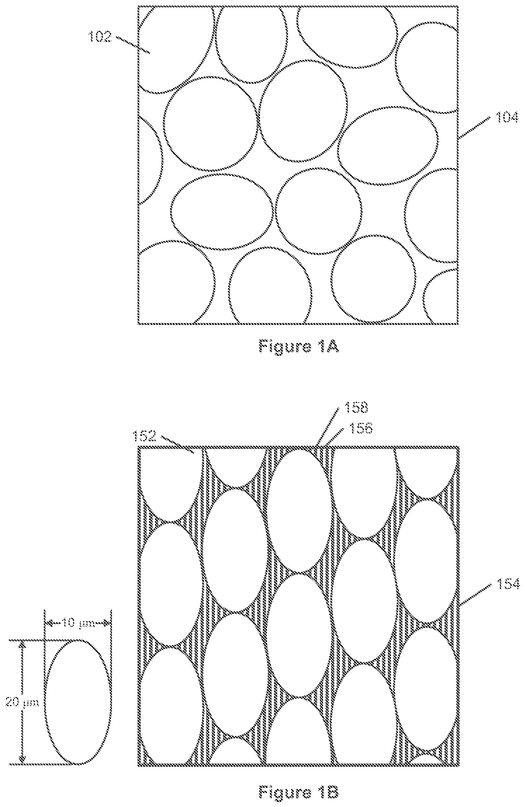

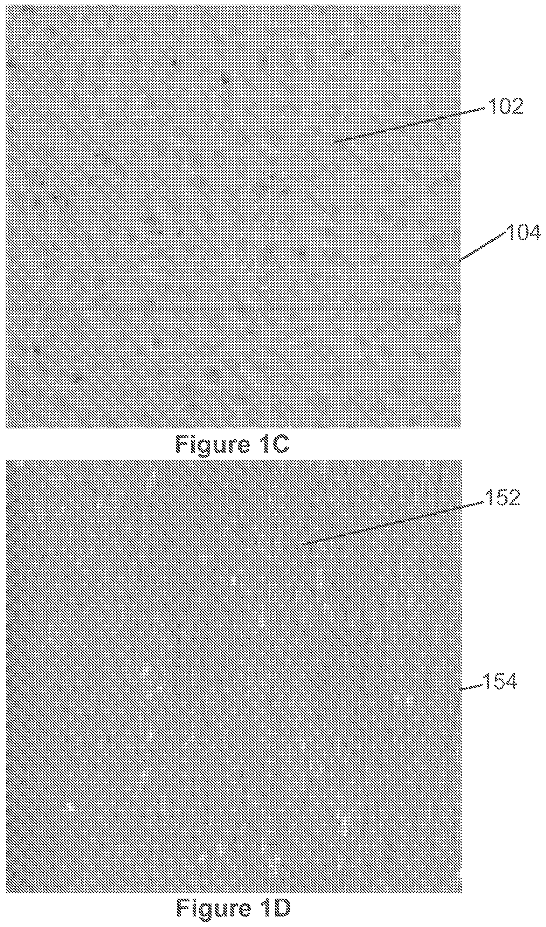

The effect of an exemplary surface topography on kidney cells is shown in Figure 1. Figures 1A and 1B show two diagrams of kidney cell cultures. Figures 1C and 1D show two photographs taken of kidney cell cultures. Figure 1A is a diagram of a culture of kidney cells 102 grown on a flat surface 104, such as a typical culture plate, dish, or flask. Traditionally, kidney cells are cultured in a fluid static state, experiencing no shear stress or topography. Even if the kidney cells experienced fluid flow and shear stress, the cells would not align and their morphology may not be affected. As seen in Figure 1A, typical cultured kidney cells 102 do not have a characteristic shape or alignment, but rather appear randomly aligned. Additionally, the cells 102 do not form superstructures and are not typically joined to each other. Figure 1C is a photograph of a culture of kidney cells gown on a flat surface 104. Figure 1C illustrates how the cells 102 lack a characteristic shape and align randomly.

Figure 1B is a diagram of a culture of kidney cells 152 grown on a surface 154 with an exemplary topographical pattern. The surface has grooves 156 and ridges 158 that are narrower than the cells 152. Each cell 152 straddles several grooves 156 and ridges 158. This pattern of grooves and ridges, like the extracellular matrix (ECM), causes the kidney cells 152 to lengthen and align themselves parallel to the ridges, encourages cell-to-cell junctions, and promotes the adhesion of the cells 152 to the surface 154. The scale given on the left gives approximate dimensions for some epithelial cells of the kidney; however, the actual sizes of kidney cells vary widely throughout the nephron. The morphology of the cells 152 is also exemplary; some types of kidney cells, particularly columnar cells, are more rectangular. In Figure 1B, the grooves 156 and ridges 158 are approximately the same width, although they do not have to be. For a kidney cell with an elongated width of about 10 $\mu$m such as the cells 152, the width of the grooves 156 and ridges 158 should be about 5 $\mu$m or less so that the elongated cell is in contact with more than one ridge. In Figure 1B, the widths of the grooves 156 and ridges 158 are about 800 nm. The grooves 156 and ridges 158 may be narrower than this, but if they are narrower than about 20 nm, the texture would have limited effect on kidney cells. The sizing of the topographical pattern depends on the size of the cells, however. Furthermore, for certain applications, it is desirable to have wider grooves in relation to the cell width so that the cells rest in the grooves. In such an aspect, the ridges may be narrower than the grooves. In other aspects, the ridges may be wider than the grooves.

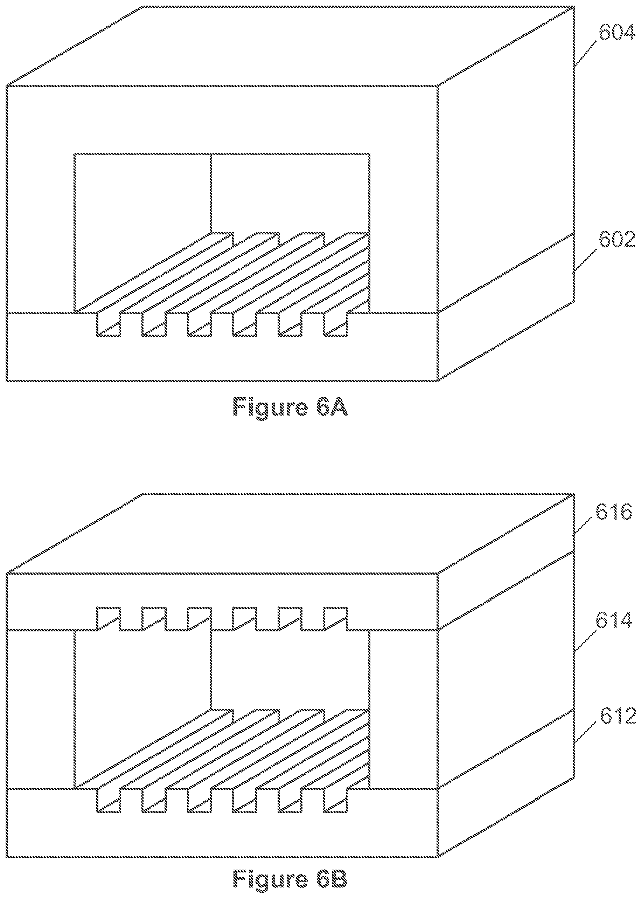

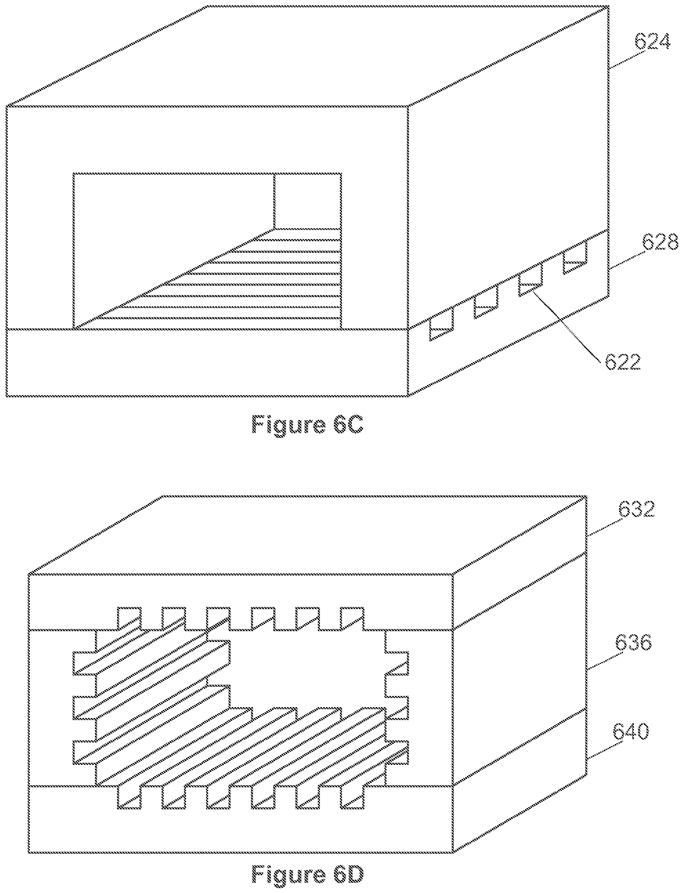

In addition to controlling the orientation and shape of the cells, the grooves and ridges also cause the kidney cells 152 to join together and form more defined and well-developed junctions as compared to cells grown on a non-textured or "blank" substrate for the same period of time. The tight cell junction observed *in vivo* is necessary for nephrons to filter blood and reabsorb water properly. The tight junctions prevent fluid, including liquid and dissolved solutes, from passing in between cells, so fluids exiting the walls of the nephron must pass through the epithelial cells, which can control which fluids and/or solutes are passed. These cell junctions also will form in an *in vitro* environment when a surface topography, such as the grooves 156 and ridges 158, causes confluent cells to align so that their membranes can join to form this fluid-impermeable barrier. Flow channels can be arranged in any direction in relation to the surface 154 to cause fluid to flow in any direction over the cells 152. In embodiments shown in Figure 6, fluid is flowed either parallel or perpendicular to the grooves 156 and ridges 158.

Figure 1D is a picture illustrating the results of one possible aspect of the invention. Figure 1D shows a kidney cell culture grown on a substrate with a topological texture 154. As in the illustration, Figure 1B, the cells 152 appear to grow in a more aligned and tightly connected manner.

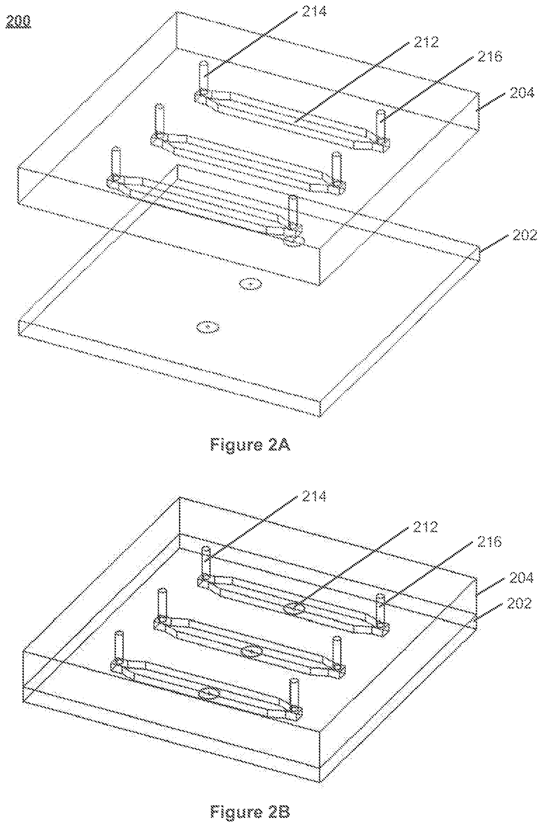

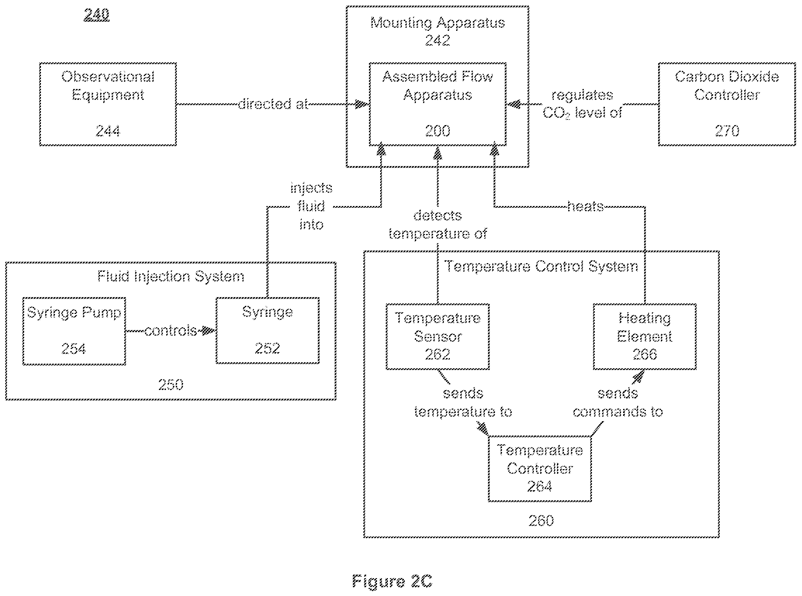

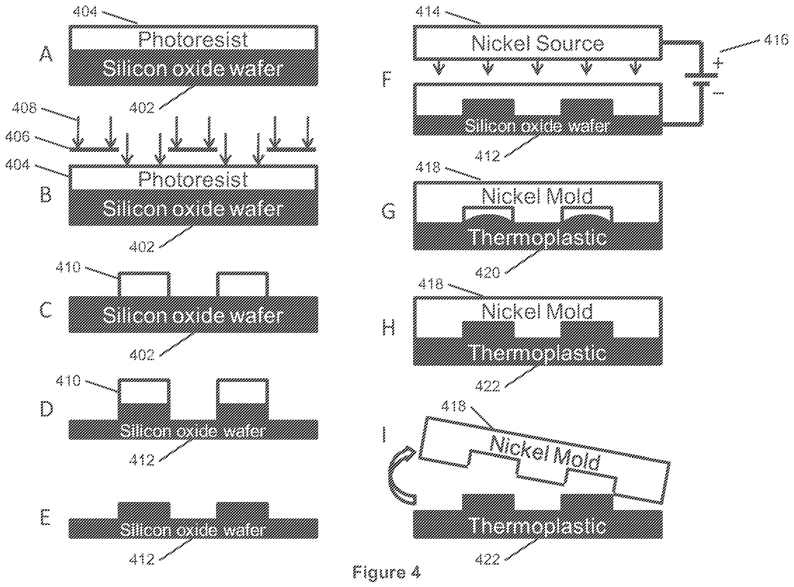

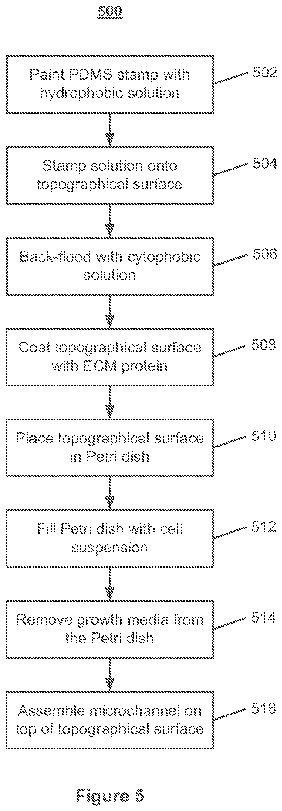

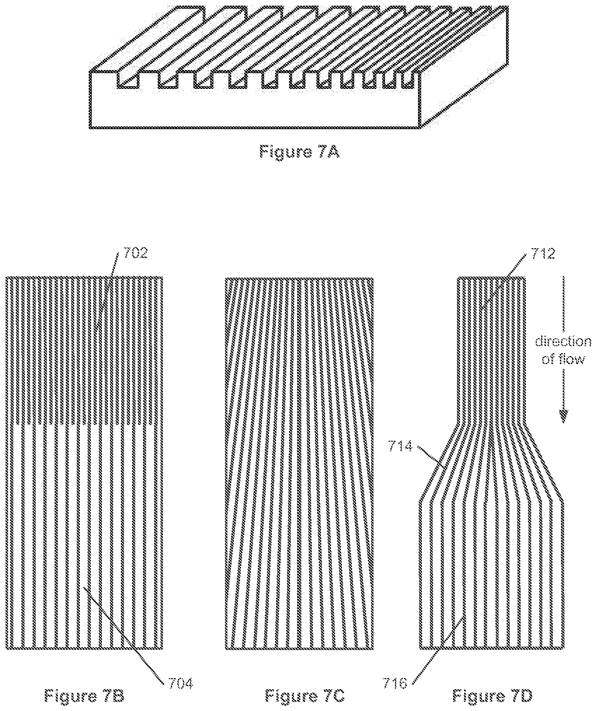

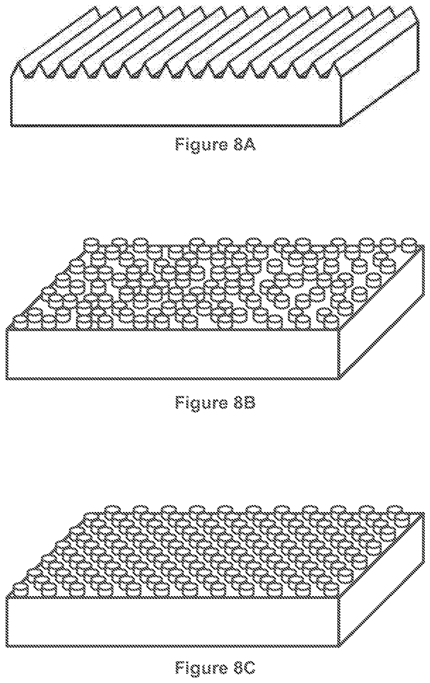

Figure 2A is an exploded solid model showing an exemplary aspect of a device 200 for culturing cells in a biomimetic environment. Figure 2B shows the assembled device. The device 200 includes a substrate 202 and a flow cell 204 having three flow channels 212. The top of the substrate 202 have a topographical pattern not shown in Figure 2, such as the pattern of Figure 1B. The walls of the flow channels 212 may also or alternatively have a topography, such as the pattern of Figure 1B. Atop the surfaced side of the substrate 202 is a cell layer, with cells preferably confined to the area of the substrate underneath the flow channels 212 in the flow cell 202. The substrate 202 can be made of a thermoplastic, such as polystyrene or polyimide, biodegradable polyesters, such as polycaprolactone (PCL), or soft elastomers such as polyglycerol sebacate (PGS). The substrate 202 may alternatively be made of polydimethylsiloxane (PDMS), poly(N-isopropylacrylamide), or nanotubes or nanowires formed from, for example, carbon or zinc oxide. The substrate 202 can be made of any material upon which a micron-scale topography can be formed and upon which cells can be grown. Examples of topographical patterns are shown in Figures 7A-7D and Figures 8A-8F, and a method for chemically-patterning the substrate 202 is described in relation to Figure 4. A method for growing the cell layer is described in relation to Figure 5.

The flow cell 204 has three flow channels 212 cut into the flow cell 204 from the bottom. In some aspects, the flow cell is made of a translucent material such as PDMS. Alternatively, the flow cell may be made of other materials, such as any of the possible substrate materials listed above. A 3D solid object printer or photolithography may be used to create the flow channels 212 in the flow cell 204. As shown in Figure 2B, when assembled, the substrate 202 forms the bottom wall of the flow channel, and the flow channel 212 in the flow cell contains the top wall and two side walls. The top, bottom, and side walls are collectively referred to as "walls" herein. The flow cell 204 and substrate 202 may be reversibly attached, so that after an experiment has been run, the flow cell 204 can be removed so that the cell layer can be examined. An inlet 214 and an outlet 216 allow tubing access to the flow channel 212. The inlet 214 provides access for introducing a fluid into the flow channel 212, and the outlet 216 provides access for removing fluid that has passed through the flow channel 212. The tubing that enters the inlet 214 connects to a means of controlling injection volume and speed into the flow channel 212, such as a syringe with a syringe pump. The fluid injection system may be part of a biomimetic flow system that the apparatus 200 is mounted in. A block diagram of an exemplary flow system 240 is shown in Figure 2C. The flow system may contain additional features, such as temperature control system and observational equipment. The fluid flow introduced through the inlet port 214 creates a uniform laminar flow along the walls of the flow channel 212 and over the cultured cells along the bottom of the channel.

The laminar flow across the cultured cells mimics the flow of fluid within a nephron and introduces a shear stress between the fluid and the cultured cells. Adjusting the flow rate of the fluid causes the shear stress along the cells to change. In a renal proximal tubule, cells experience fluid shear stress (FSS) of around 1 dyn/ $cm^2$; however, the shear stress experienced varies along the length of a nephron. Shear stress has been observed as low as 0.015 dyn/$cm^2$, so being able to vary the shear stress by controlling the flow rate allows a range of biomimetic conditions to be produced with a single device design. For a rectangular channel with a width w and height h, and a fluid with a viscosity $\mu$, the wall shear stress $\tau$ and flow rate Q have the following relationship:

$$\tau = \frac{6\mu Q}{bh^2}$$

In addition to varying the shear stress of a channel by changing the flow rate, the shear stress in a single channel can vary along the length of the channel if the geometry of the channel changes along the length of the channel. For example, in a flow channel having a first height in a first region of the channel along the direction of the flow and a second, lower height in a second region of the channel, the fluid flowing in the shorter second region of the channel has a higher flow rate than in the taller first region. Thus, the cells in the second region experience a higher shear stress than the first region. By smoothly varying the channel height, the cell layer experiences a gradient in shear stress. The varying shear stresses can be used for replicating different parts of a nephron, which may have different functions. Additionally, different types and arrangements of cells are associated with regions of the nephron that experience different levels of shear stresses, so regions along the substrate having different types or arrangements of cells can be lined up with a flow channel of varying height so that cells experience the appropriate shear stress.

The flow channels 212 in Figure 2 have rectangular cross sections, but flow channels may have non-rectangular cross sections for different fluid dynamics. For example, the flow channel may have a semicircular, triangular, or trapezoidal cross section. The shear stress can be determined using the shear stress equation for the cross section geometry. The flow channel may have two or more different cross section shapes along its length. Other mechanisms and arrangements can be used to vary the shear stress along the channel. For example, more complex flow patterns may be produced by linking multiple channels in a network. The shear stress experienced by cells along each channel results from the network flow patterns.

The assembled flow apparatus 200 may be placed in a flow system 240, shown in the block diagram of Figure 2C, for holding the assembly and controlling the flow channel environment. The flow system includes a mounting apparatus 242, observational equipment 244, a fluid injection system 250, and a temperature control system 260.

The mounting apparatus 242 includes a plastic cover and plastic base made using, for example, a 3D solid object printer. The flow channel apparatus 200 is positioned between the cover and base of the mounting apparatus 242 which are held together by, for example, screws or clamps. Thus, the mounting apparatus 242 holds the substrate 202 and flow cell 204 of the apparatus 200 together, preferably forming a reversible seal so that no fluid can leak out of the flow channel 212.

The observational equipment 244 is used to observe the cells during and/or after the experiment while the flow channel apparatus 200 is held in the mounting apparatus 242. If a wall of the channel is transparent, an optical microscope can be used to observe the sample during the experiment. In some aspects, the observational equipment includes a light source directed into the flow channel and optical sensors underneath the flow channel for detecting extraordinary optical transmission (EOT) signals. In such an aspect, both the flow cell 204 and substrate 202 should be made of a material that allows optical transmission. In other aspects, electrodes are placed in the flow channel for detecting confluence, shape, and metabolism of the cells. In some aspects, imaging is performed outside of the mounting apparatus, possibly after the flow channel apparatus 200 has been disassembled, and the flow system 240 does not have observational equipment 244. The observation equipment can include, but is not limed to, sensors for the monitoring of temperature, oxygen levels, pH, carbon dioxide, and fluorescent tags and other markers.

The fluid injection system 250 includes a syringe 252 and a syringe pump 254. The syringe 252 contains the fluid to be flowed through the flow channel 212, and the syringe pump 254 controls the syringe 252 to control the amount of fluid flowed, the flow rate, and the length of time that fluid is flowed. The fluid injection system 250 can be used to inject two or more different fluids, including, but not limited to, a buffer fluid, a reactant fluid, a fixing solution, and a stain. The fluid injection system 250 may also include means to combine the fluids; for example, the syringe pump 254 may include a valve for introducing a reactant into the buffer solution, or for introducing a stain into the buffer solution. The syringe pump 254 may be controlled by a processor, such as a general purpose processor.

The substrate 202 and/or flow cell 204 may be made of an insulating material to maintain a temperature stability within the flow channels 212. The flow system 240 can include a temperature control system 260 or incubator. The temperature control system includes a temperature sensor 262, a temperature controller 264, and a heating element 266 to maintain the flow channels 212 at a biomimetic temperature, *i.e.,* 98.6° F. The temperature can be selected to be higher or lower to simulate different conditions, e.g., fever or injury. The temperature 262 sensor detects the system temperature and sends it to the temperature controller 264. Based on the current system temperature, the temperature controller 264 determines if heat should be added to the system. The temperature controller 264 sends commands to the heating element 266, such as a thermistor, to add heat to the system as necessary. In some aspects, the temperature system can also cool the apparatus 200. The temperature controller or a second temperature controller also maintains the temperature of the fluids to be flowed into the flow channels 212. The flow system 240 also can include a carbon dioxide controller 270 to regulate the carbon dioxide level inside the flow channel. The carbon dioxide controller 270 can include a carbon dioxide sensor, a processor, and means for increasing or decreasing carbon dioxide levels as necessary. In some aspects, the carbon dioxide controller is integrated into the temperature control system 260.

In some aspects, the flow system 240 is connected to a computer with a general purpose processor. The computer can act as temperature controller 264, and temperatures from the temperature sensor 262 are sent to the computer and saved to memory. Similarly, the computer can act as a carbon dioxide controller, and carbon dioxide levels from a carbon dioxide sensor can be sent to the computer and saved to memory. The computer can also control the syringe pump 254 to automate the fluid flow and save data about the fluid flow. The computer can also control the observational equipment, save images or other data obtained from the observational equipment, and perform analysis on the data.

The apparatus 200 may contain more or fewer flow channels 212 than three. A high-throughput apparatus may be produced containing a hundred or more flow channels 212. In some aspects, the high throughout apparatus contains ninety-six flow channels. The inlets 214 of two or more flow channels 212 may be fluidly connected so that fluid is flowed through multiple flow channels 212 simultaneously and, if the geometries of the flow channels 212 are identical, at the same flow rate. The flow system 240 may be configured to automatically run multiple experiments at the same time or perform experiments one at a time. An experiment performed in one flow channel in the apparatus 200 could differ from an experiment performed in a different flow channel in at least one of a fluid, a flow rate, the flow channel geometry, and the temperature. The apparatus 200 may be disposable or, after use, all or a portion of the apparatus (*e.g.,* the substrate 202 or the flow cell 204) may be disassembled and cleaned for reuse.

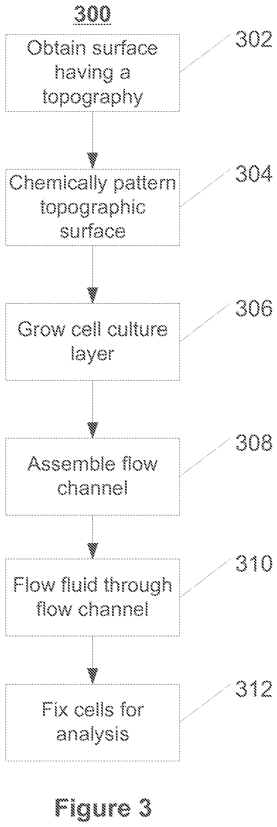

Figure 3 is a flowchart for a method 300 of creating and using one of the biomimetic flow channels shown in Figure 1. The method 300 includes the steps of obtaining a surfaced substrate (302), chemically patterning the substrate (304), growing a cell layer (306), assembling the flow channel (308), flowing fluid through the flow channel (310), and fixing the cells for analysis (312).

The first step 302 is to produce or obtain a surface with a topographical pattern such as the grooves 156 and ridges 158 on the surface 152 of Figure 1B. The surface may be created using, for example, direct lithography, photopatternable resists, injection molding, direct micromachining, deep RIE etching, hot embossing, or any combinations thereof. An exemplary method for producing a surfaced substrate by hot embossing for use in the apparatus 200 is described in relation to Figure 4. In some aspects, a thin layer of metal, such as gold, is evaporated onto the topographical surface to aid in optical imaging for analyzing the sample. In some aspects, the metal layer allows analysis of the cell culture using optical sensors for detecting surface plasmon resonance (SPR). In some aspects, the metal layer is also useful to chemically pattern certain cytophilic and cytophobic molecules on the substrate due to the strong affinity of the molecular head group to the metal. The metal layer is thin enough so that the topography of the surface is maintained. The metal layer may have nanoholes for use in SPR detection. The nanoholes may be on the order of 100 nm wide and milled into the metal using, for example, photolithographic techniques, electron beam lithographic techniques, a focused ion beam (FIB), or other methods. The metal layer could alternatively be made of silver, aluminum, beryllium, rhenium, osmium, potassium, rubidium, cesium, rhenium oxide, tungsten oxide, copper, titanium, or another material suitable for imaging or other analysis. In some aspects, a thin layer of chromium or other metallic bonding agent is first evaporated onto the surface before the layer of gold or other metal.

Next, the textured surface is chemically patterned (step 304) to produce cytophobic and/or cytophilic regions for isolating cell growth to the flow channel areas. If kidney cells are grown across an entire surface, such as substrate 202, then sealing the flow cell 204 to the substrate 202 would crush the cells in the regions outside of the flow channel 212. This creates leakage of contents from the crushed cells into the flow channel 212, which can provide biochemical signals received by the surviving cells. Such leakage could have undesirable effects on the experimental procedure. Thus, cells should be restricted to the regions of the surface that form the walls (*i.e.,* top, bottom, and/or sides) of the assembled flow cell. For the apparatus 200, the cell layer is restricted to the regions of the substrate 202 that do not contact the flow cell 204, *i.e.,* the regions below the flow channels 212. To create these regions, cytophilic materials can be placed on the flow channel surfaces to promote cell growth for regions within the flow channel. Additionally or alternatively, cytophobic materials can be placed on surfaces which do not form a wall of a flow channel to prevent cell growth outside of the flow channel surface. Once the topographical surface has been created and chemically patterned, an ECM protein is applied to the surface and a cellular suspension is placed above the protein layer to grow a cell culture (step 306), which contains one or multiple types of cells. One method for creating cytophilic and cytophobic regions of a surface, such as substrate 202, and growing cells on the surface is described in relation to Figure 5.

Once the cells are grown (step 306), the flow channel is assembled (step 308). In some aspects, the flow cell is reversibly assembled so that at least one wall can be removed for observation. In some aspects, the flow cell 204 is placed on top of the substrate 200 and attached using, for example, screws or clamps. Alternatively, the apparatus 200 may be assembled within the mounting assembly 242 as described in relation to Figure 2C.

A fluid is then flowed through the assembled flow channel (step 310). The flow rate is selected to create the desired shear stress as described in relation to Figure 2, and the flow rate is controlled by the fluid injection system. The fluid may contain a reagent and a buffer fluid. The reagent is chosen based on the experiment being performed; for example, the fluid may include a pharmaceutical agent or a potential toxin to test the efficacy or toxicity of the substance. The fluid may be flowed through the flow channel for any duration, for example, on the order of seconds, minutes, hours, or days. The duration of fluid flow depends on the type of experiment being performed. For example, an experiment for imaging the cells experiencing a particular shear stress may only last a short duration, on the order of tens of seconds to several minutes. On the other hand, an experiment for determining the effect of a potential toxin or a potential therapeutic agent on the cells may require a longer exposure time, on the order of several hours, several days, or longer. The flow rate may vary over time, and in some aspects, the cells may experience intermittent fluid flow.

Next, if post-flow analysis is to be performed, the cells are fixed for analysis (step 312). First, the cells may be rinsed by flowing a second solution, such as phosphate buffered saline (PBS), through the flow channel. Then, a third solution containing a fixative, such as formaldehyde or other aldehyde, is flowed through the channel so that the channel can be disassembled without damaging the cells. The percentage of fixative in the solution and the type of fixative chosen depends on the method of analysis and the type of cell in the flow channel. The rinsing solution and fixative are injected through the inlet 214 using the fluid injection system. The same injection mechanism, such as the syringe pump, and tubing may be used and the syringe swapped out, or the mounting apparatus may contain at least two or three syringe pumps connecting via tubing to the same inlet. A stain may also be applied to the cells before or after disassembling the flow channel to increase contrast for imaging.

Figure 4 is a series of diagrams illustrating a method for producing a surface with topographical patterning such as surface 102 from Figure 1. The method involves etching a silicon oxide master mold (steps A-E), electroforming a negative nickel mold (step F), and using hot embossing to produce the topographical surface from the nickel mold (steps G-I).

The silicon oxide master mold is created using photolithography techniques. Step A illustrates coating a photoresist solution 404 onto a silicon oxide wafer 402. The photoresist 404 can be applied using spin coating. After spin coating, the silicon oxide wafer 402 may be baked to evaporate residual solvent. A photo mask 406 is then layered on top of the photoresist 404 (step B) to photolithographically pattern the resist 404. In Figure 4, the photo mask 406 has the cross-section of a ridge pattern. The pattern of the topography is determined by the photo mask selected. Alternative topographical patterns, which would be made with different photo masks, are shown in Figure 6. The photoresist 404 is developed to create an etch mask 410 (step C) by exposing the top surface to light 408. In Figure 4, a negative photoresist 404 is used, so the portion of the photoresist layer 404 that is exposed to light 408 becomes insoluble in the developer, while the unexposed portion is soluble and is dissolved when developed. Alternatively, a positive photoresist can be used. Once the pattern has been generated in the photoresist 404 to produce the etch mask 410, the silicon wafer may again be baked, and the wafer 402 is developed to remove the photoresist 410 from the areas exposed to the light. Finally, the silicon oxide wafer 402 is etched using a chemical agent that removes the uppermost layer of the wafer not protected by etch mask 410 (step D). In some applications, the depth of silicon oxide removed is on the order of 1 micron. To produce a pattern with square edges as seen in Figure 4, an anisotropic etchant is used; if rounded wells are desired, an isotropic etchant can be used. Finally, the etch mask 410 is stripped to create the master mold 412 (step E).

Other methods can be used to create the master mold 412. For example, electron beam lithography or other nanolithography techniques can be used to etch features into the resist, particularly if the desired topography includes very small-scale features with widths on the order of nanometers or tens of nanometers.

Once the master mold 412 has been created, nickel 414 is electroformed to the silicon wafer (step F). A nickel source 414 and the master mold 412 are placed in an electrolytic bath. A power source 416 creates a voltage difference between the nickel source 414 and the silicon oxide master mold 412 that causes nickel to electroplate onto the master mold 412 to form a nickel mold 418 that is a negative of the master mold 412. The nickel mold 418 is separated from the master mold and placed face down and pressed against a thermoplastic 420. The thermoplastic is pushed into the grooves in nickel mold 418 through hot embossing (step G). Elevated heat and pressure causes the thermoplastic 420 to fill into the nickel mold. Then, the embossed thermoplastic 422 is cooled under constant pressure (step H) before being removed from the nickel mold 418 (step I). The topographical surface in the molded thermoplastic 422 can then be used as a wall of a flow channel.

Figure 5 is a flowchart for a method of creating cytophilic and cytophobic regions of a surface and growing cells on the surface, according to an illustrative aspect of the invention. The method involves creating a hydrophobic region on a surface for the area that will form a wall of a flow channel (steps 502 and 504), filling in the rest of the surface with a hydrophilic substance (step 506), coating the hydrophobic region with a protein (step 508), and growing a cell layer atop the protein, *i.e.,* the cytophilic region (steps 510 and 512). In general, extracellular matrix (ECM) proteins adhere to hydrophobic molecules. Cells thrive in conditions similar to *in vivo* conditions, so cells tend to grow in regions containing ECM proteins rather than hydrophilic regions that do not contain ECM proteins.

First, a hydrophobic solution is applied to a PDMS stamp (step 502) and stamped onto a surface having a topography (step 504). The stamp is held with pressure and released so that a hydrophobic self-assembled monolayer (SAM) of molecules remains on the surface. Molecules used for a hydrophobic SAM typically have a head group that binds to the substrate and a hydrophobic tail group, such as $CH_3$, that binds to ECM proteins. In some aspects, hexadecanethiol is used for the SAM. Other molecules that can be used for the SAM include alkyl thiols, functionalized thiols, dithiols, and silanes.

The painted surface of the PDMS stamp can be the size of the side of the flow channel being stamped, or the painted surface could only cover the area of the flow channel to be filled with cells. For example, if it is not desired to have cell growth near the ends of the channel by the fluid inlet and outlet, a rectangular stamp to cover only the central part of the flow channel would be used. For an apparatus with multiple flow cells, a single PDMS stamp may have a pattern for stamping the walls of multiple flow channels.

The rest of the surface is back-flooded with a hydrophilic solution, such as polyethylene glycol (PEG), to form a SAM that discourages cell growth on the parts of the surface which will not be flow channel walls (step 506). As described in Figure 1, this keeps cells from growing outside of the desired area to prevent leakage from cells crushed when the apparatus is assembled as well as unwanted edge effects, such as negative communication between crushed cells and cells within the channel.

The topographical surface with the two SAMs is coated with an extracellular matrix (ECM) protein, 3-D peptides, synthetic proteins, and synthetic adhesive motifs, or combinations thereof (step 508). The protein adheres to the hydrophobic SAM, but the hydrophilic SAM suppresses protein adsorption. This causes the protein to concentrate in the hydrophilic region that will form a wall of the flow channel when the apparatus is assembled. In some aspects, different types of cells may be grown in different regions of a flow channel or on different flow channels having walls formed on the same surface. To isolate cell types to certain regions of the surface, the surface may be treated with different proteins and/or SAM molecules to promote growth of certain cell types in certain regions. Other methods can be used to create a cytophilic region. For example, a thermopolymer substrate can be treated with an oxygen plasma to increase cytophilicity, and any untreated region will be cytophobic.

The treated surface is then placed in a Petri dish to grow the cells (step 510). The Petri dish is filled with a cell suspension solution and placed in an incubator set at 5% $CO_2$ and 37° C in some aspects (step 512). Cells adhere only to the protein-coated cytophilic region. The cells are cultured to a high confluency so that the when assembled, the flow channel wall will be covered in a layer of cells. Cells that are commonly grown for *in vitro* kidney models include human proximal tubule cells, such as cells from the HK-2 line; renal proximal tubule epithelial cells (RPTEC); Madin-Darby canine kidney (MDCK) cells; and primary inner medullary collecting duct (IMCD) cells or primary proximal tubule cells, commonly collected from mice or rats. In various aspects, stem-cell derived cells, kidney progenitor cells, or cells harvested from kidney tissue are grown. Cells from humans, other mammalian organisms, or other organisms may be grown.

Once the cells are grown, the media in which the cells were grown in was removed (step 514). Typically, the media is removed from the areas outside of the cell layer. Thus, some moisture remains within the region with the cell layer, but the rest of the substrate is dry. Then, the flow microchannel is assembled atop the topographical surface (step 516). Once the flow microchannel is on top of the topographical surface, the microchannel can be filled with the cell growth media.

Figure 6A shows a perspective view of a cross section of an illustrative aspect of a biomimetic flow channel with ridges and grooves parallel to the direction of the flow channel. The ridges and grooves may not be to scale. As described in relation to Figure 1, the width of the ridges and grooves may be in the range of 20 nm to 5 microns. For example, the width of the ridges and/or grooves may, independently, be 20-40 nm, 30-50 nm, 50-100 nm, 100-200 nm, 200-400 nm, 400-600 nm, 600-800 nm, 800 nm - 1 micron. By way of further example, the width of the ridges and/or grooves may be, independently, 1-2 microns, 2-3 microns, 3-4 microns, or 4-5 microns. The height of the flow channel may be in the range of 100 to 200 microns. Larger or smaller heights can be used, but if a flow channel is much narrower than 100 microns high, fluid flowing through the channel may not be in laminar flow. In the aspect shown in Figure 6A, the cell layer would be grown above the bottom layer 602 of the flow channel, and the ridges and grooves would cause the cells to align parallel to the direction of flow through the channel.

In the aspect shown in Figure 6B, both the bottom surface and the top surface are textured and cells are cultured on both, which may better replicate an *in vivo* nephron or another organ. The bottom layer 602 in Figure 6A ,the top and bottom layers 612 and 616 in Figure 6B, and layers 632, 636, and 640 in Figure 6D may be topographical substrates such as substrate 202 of Figure 2. In some aspects, layer 616 in Figure 6B has at least two holes through it so that fluid can be flowed into the flow channel through one hole and removed through the other. In Figure 6B, a middle layer 614 having an elongate hole formed therethrough is sandwiched between the top and bottom layers 616 and 612. When positioned between the top layer 616 and bottom layer 612, the side walls of the elongate hole form the side walls of a flow channel.

Figure 6C shows a perspective view of a cross section of another illustrative aspect of a biomimetic flow channel which has ridges and grooves perpendicular to the direction of the flow channel. The ridges and grooves may not be to scale. The dimensional ranges for the topography and flow channel of Figure 6C are the same as the ranges given in relation to Figure 6A. The cell layer would be grown above the bottom layer 622 of this flow channel, and the ridges and grooves would cause the cells to align perpendicular to the direction of flow through the channel. The top surface and/or side surfaces of the channel may also be textured and have cells grown thereon (not shown).

As can be seen at the right side of Figure 6C, the entire top surface of the lower layer 622 is textured with ridges and grooves. Since the sides of the flow channel may not be sealed, fluid flowing through the flow channel may seep out of the sides of the flow channel through the holes 628 created by the grooves. So, in some aspects, the topographical surface is created only on the part of the surface that will form a wall of a flow channel; the rest of the surface is smooth. This can be accomplished by using a photo mask that completely covers the surface of the substrate except for the regions that will be flow channel walls. When the flow channel is assembled, the smooth surface joined to the flow cell or side walls would create a seal at the sides of the flow channel along its length, preventing fluid from travelling sideways out of the ridges and grooves.

Figure 6D illustrates a perspective view of another illustrative aspect of a biomimetic flow channel. In this aspect, the bottom, top and both side walls are textured and cells are cultured on each, which may better replicate an *in vivo* nephron or another organ. In this illustrative aspect, the texture consists of ridges and grooves parallel to the direction of the flow channel. In some aspects, layer 632 in Figure 6D has at least two holes through it so that fluid can be flowed into the flow channel through one hole and removed through the other. An elongated hole is formed when the walls 636 are sandwiched between the top 632 and bottom 640 layers. The side walls of the elongated hole form the side walls of a flow channel.

Figure 7 shows several embodiments of topographical patterns having variation in ridge and/or groove width. Figure 7A is a perspective view of a substrate having a gradient in its topographical pattern. The widths of the ridges and grooves become narrow from left to right across the surface. A flow channel may be assembled atop the substrate to cause fluid to flow either parallel or perpendicular to the grooves. In some embodiments, the widths of the ridges stays constant while the widths of the groove changes; in other embodiments, the widths of the grooves stays constant while the widths of the ridges varies. A surface with variations in topography can be used in an experiment for examining the effect of different ridge and/or groove widths on a particular type of cell. Additionally, for an organ structure that has varying cell features across an area of the structure *in vivo,* smoothly varying surface topography as in Figure 7A can be used to create a flow channel that mimics such a feature.

Figures 7B, 7C, and 7D are top views of flow channel surfaces having variations in their topographical patterns along the channel. The flow channels may not be to scale. The direction of flow across the channels is down the page, as shown by the arrow to the right of Figure 7D. In other embodiments, fluid can be flowed in the opposite direction. Figure 7B is a top view of a surface with two distinct regions 702 and 704 having different groove widths. The upper region 702 has a narrower groove width so that the grooves and the ridges are about the same width. The lower region 704 has noticeably wider grooves than the upper region 702. The grooves in the lower region 704 are also wider than the ridges. A flow channel having two distinct topographical regions can be used to mimic an *in vivo* structure that has two distinct cell types or cell arrangements. For example, a kidney tubule has cell types that change along its length, and variations in the basement membrane of a kidney tubule can be simulated *in vitro* by varying the topography of the flow channel.

The grooves of Figure 7C also are wider towards the end of the flow channel; however, the ridges fan out so that the grooves widen smoothly, creating a smooth transition between the narrow grooves and the wider grooves along the channel rather than distinct regions. A flow channel with one or more sides of this type can be used to mimic an *in vivo* structure that has a gradient in cell type or cell arrangement across the structure. A flow channel with this type of topography could also be used in an experiment for examining the effect of different ridge and/or groove widths on a particular type of cell.

Figure 7D is a top view of a non-rectangular channel having two regions of constant topography 712 and 716 that differ from each other and a transition region 714 between the two regions of constant topography. The start of the flow channel 712 has a narrow width and narrow grooves, and the end of the flow channel 716 is wider and has wider grooves. All of the regions 712, 714, and 716 have the same number of ridges. If the flow channel atop the surface of Figure 7D has a constant height, the shear stress experienced in the first region 712 will be higher than the shear stress in the last region 716 since the channel width is smaller and the flow rate is higher in the first region 712 than in the last region 716. This effect can alternatively be achieved by varying the channel height. The transition region 714 helps maintain laminar flow as the geometry changes. The methods of manufacture described in Figure 3, 4, and 5 can be used to create flow channel devices with any of the topographies and geometries shown in Figure 7.

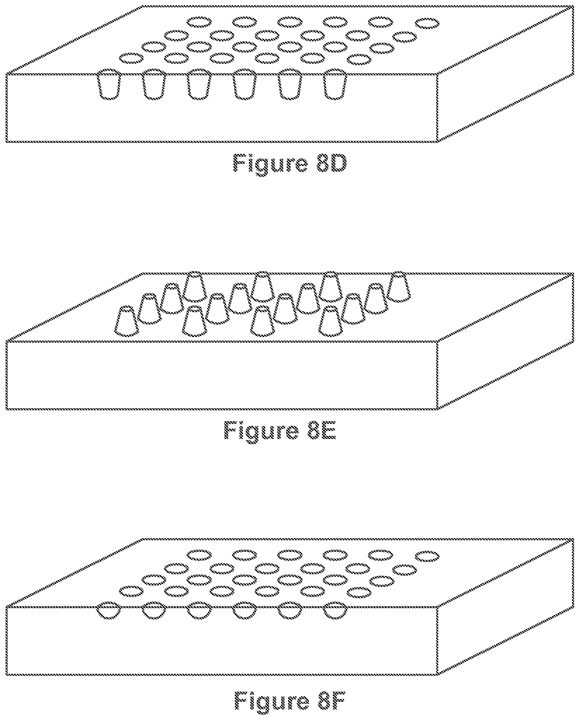

Surface topographies can have patterns other than the right-angled ridges and grooves shown in Figures 6 and 7. Some possible topographic surfaces are shown in Figures 8A through 8F, which are perspective views of six substrates with different topographical patterns. Figure 8A shows triangular ridges separated by narrow grooves. The edges can be more rounded than are shown in Figure 8A. Figure 8B shows a random array of circular posts. Figure 8C shows a linear array of posts. Figure 8D shows a linear array of inverted cone pits. Figure 8E shows a linear array of cone posts. Figure 8F shows a linear array of inverted hemisphere pits. Any of the textures that can be broadly categorized as pits and posts can be arranged into random or linear arrays. Additionally, Figure 8 is not intended to encompass all possible designs of pits and posts but rather provide an illustration of possible designs. The pit and post family may also include, but is not limited to, posts, cones, hemispheres, filleted pits, and chamfered posts. The topography selected depends on the type of cells being cultured, as different types of cells may have different *in vivo* environments and may respond differently to biomimetic cues. While ridges and grooves have been shown to provide biomimetic cues causing kidney cells to align, the extracellular matrix differs in different parts of the body, so cells in different organs or even other parts of the kidney may better mimic their *in vivo* characteristics when grown on the types of topographies shown in Figure 8 or another type of topography. A method similar to the method described in relation to Figure 4 can be used to form the substrates shown in Figure 8A - 8F. Other techniques may be used, particularly for producing triangular grooves or other patterns that have grooves with neither square nor rounded edges. As described in relation to Figure 7, the size and/or separation of the features may vary along the surface, either in distinct regions or across a gradient. Additionally, a single flow channel may have multiple types of topographies, such as a region of posts and a region of ridges and grooves, or posts interspersed with ridges and grooves.

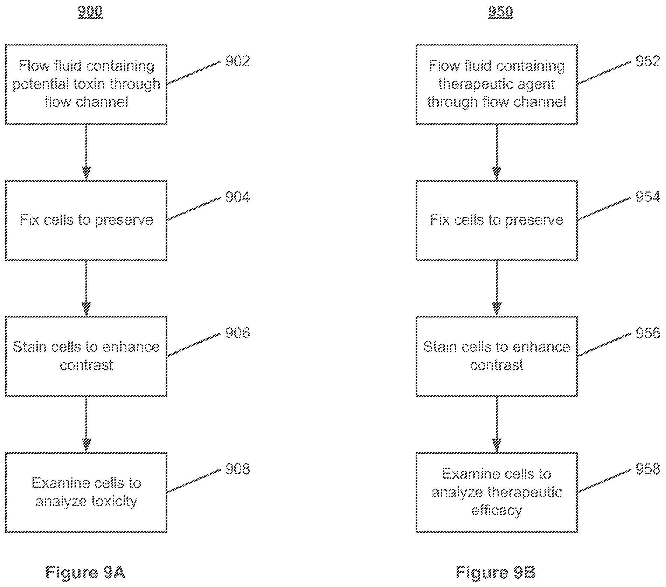

Figures 9A and 9B show two exemplary uses of biomimetic flow channels. Figure 9A is a flowchart of a method 900 for using a biomimetic flow channel, such as the apparatus 200 of Figure 2, to examine cell toxicity of a potential toxin. This method is useful in high-throughput drug screening; researchers can screen cytotoxic compounds before investing in further pharmaceutical development of the compound. For toxicity testing, a material that is potentially toxic to a type of cell is put in solution at a certain concentration and flowed through a flow channel containing a culture of the type of cells (step 902) according to the method described in relation to step 310 Figure 3. In some aspects, particularly if the potential toxin is difficult to manufacture or difficult to acquire, the injection system includes an injection valve that releases the potential toxin after a buffer fluid has established laminar flow within the flow channel. Using an injection valve minimizes the amount of potential toxin consumed in the experiment. If observational equipment is directed at the flow channel, the sample may be analyzed while the potential toxin is flowed through the channel.

After the potentially toxic solution has been flowed through the channel, the cells are rinsed and fixed (step 904) to preserve the results of the experiment for observation, according to the method described in relation to step 312 of Figure 3. A stain or fluorescent tag is then applied to the cells before or after disassembling the flow channel to increase contrast for imaging (step 906). Any staining method, such as heaematoxylin and eosin (H&E) staining, silver staining, or immunofluorescent staining that is suitable for the cell type and observational equipment can be used. In some aspects, the stain is a vital dye, such as trypan blue or propidium iodine, which cannot pass through the cell membranes of healthy cells. The vital dye is introduced into the flow channel with the potential toxin. If the compound is toxic, it may cause breakdown of the cell membranes and permit the dye to pass through the membranes and stain the cells.

Once the fluid has been flowed through the channel and the cells are prepared, the cells are analyzed to determine the toxicity of the potential toxin. In various aspects, toxicity is determined using permeability assays, cell activity assays, metabolic activity assays, or live/dead assays. If the substance is toxic, it may have caused the cells to undergo necrosis, cause the cells to stop growing and dividing, or cause apoptosis. Cells undergoing necrosis may rapidly swell, lose membrane integrity, shut down metabolism, and release their contents. As described above, the use of a vital dye can show such effects. Vital dye staining and/or other effects of cell death can be viewed using, for example, an optical microscope, an electron microscope, or a digital holographic microscope. Other techniques for determining the viability of the cells during or after the experiment can be used. A cell counter, such as a CASY counter or a Coulter counter, can count the number of viable cells. If the surface under the cells includes gold electrodes, an ELECTRIC CELL-SUBSTRATE IMPEDENCE SENSING (ECIS) based approach can be used as a sensor of the confluence, shape, and metabolism of the cells.

Figure 9B is a flowchart of a method 950 for using a biomimetic flow channel, such as the flow channel 212 of Figure 2, to examine the efficacy of a potential therapeutic agent. The method 950 can be used in high throughput screening to determine if a potential pharmaceutical compound may have a therapeutic effect. For efficacy testing, a compound that is potentially beneficial to a type of cell in a flow channel is put in solution at a certain concentration and flowed through the flow channel (step 952) according to the method described in relation to step 310 Figure 3. In some aspects, particularly if the compound is difficult to manufacture or difficult to acquire, the injection system may include an injection valve that releases the compound after a buffer fluid has established laminar flow within the flow channel. If observational equipment is directed at the flow channel, the sample may be analyzed while the compound is flowed through the channel.

After the solution has been flowed through the channel, the cells are rinsed and fixed (step 904) to preserve the results of the experiment for observation, according to the method described in relation to step 312 of Figure 3. In some aspects, a stain or fluorescent tag can be applied to the cells before or after disassembling the flow channel to increase contrast for imaging (step 906). Any staining method, such as heaematoxylin and eosin (H&E) staining or silver staining, that is suitable for the cell type and observational equipment can be used.

Once the cells are prepared, they are analyzed to determine what effect, if any, the compound has had on the cells. Reagents that bind to particular proteins or nucleic acids can be applied to the cell layer to determine the presence of the protein or nucleic acid during analysis. Physical effects of the potential therapeutic compound can be viewed using, for example, an optical microscope, an electron microscope, or a digital holographic microscope. The features of interest depend on the type of cells and the desired effect of the pharmaceutical being researched. The method for analysis should be chosen according to which cellular features a research is interested in.

**EXAMPLES**

The following examples are provided to more fully illustrate various embodiments of the present technology. These examples should in no way be construed as limiting the scope of the invention.

Example 1 - Fluid Shear Stress Enhances Alignment of Cells to Topographic Patterns

A. Biomimetic Flow Apparatus

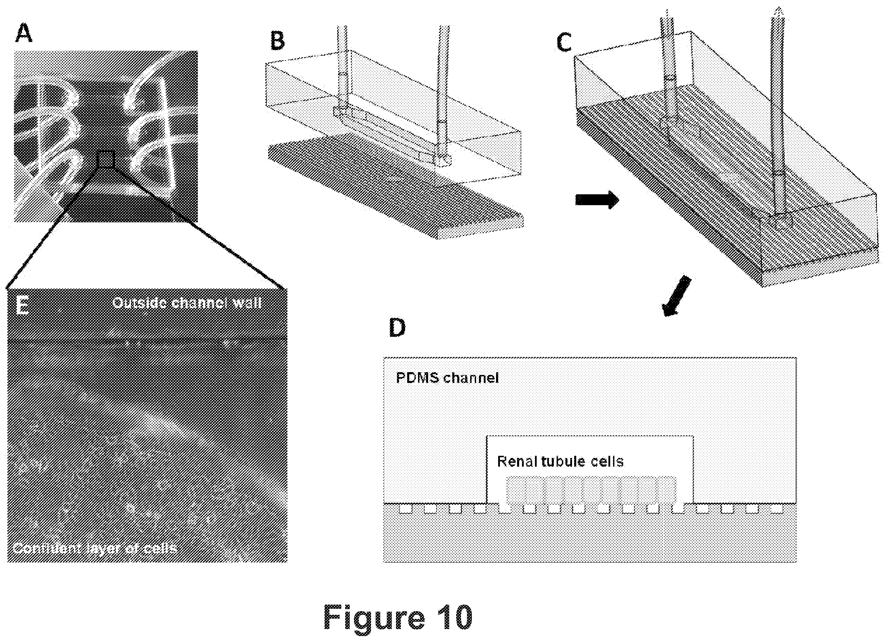

A biomimetic flow apparatus of the present technology, comprising ridge/groove features was constructed. The structure of the flow apparatus is shown in Figure 10A-D. The flow apparatus included an array of fluidic channels to control fluid shear stress (FSS) and cell substrates with controlled surface properties. The disassembled (Figure 10 B) and assembled (Figure 10 C) apparatus includes two layers: micromolded channels and a topographically patterned substrate treated with ECM protein to cue cell adhesion and function. A cross-section of the apparatus illustrates a confluent layer of renal tubule cells within the microfluidic channel and adherent to the topographical substrate (Figure 10 D). A phase contrast image of cells within the channel and adhered to the ECM-coated region (Figure 10 E).

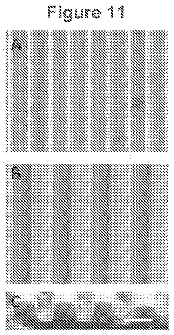

Topographical features were hot embossed from a nickel alloy mold to a polystyrene substrate (Figure 11A and B, respectively). The edge profile of the polystyrene substrate shows defined ridge/groove features (Figure 11 C). Ridges and grooves are 0.75 $\mu$m wide and 0.75 $\mu$m deep with a 1.5 $\mu$m pitch. (Scale bar, 1$\mu$m).

B. Flow-induced Shear Stress Characterization

Characterization of the flow apparatus indicated that the channel design would provide known and uniform FSS to the cell adhesion area. Computational model simulations using COMSOL Multiphysics software predict a uniform shear stress over the cell adhesion area and most of the substrate surface area (data not shown). COMSOL simulations predict uniform shear stress across cell adhesion areas and most of the cell substrate. The shear stress distribution showed higher shear stress at the inlet and outlets of the channel which quickly develops to uniform shear stress in most of the channel including the circular cell adhesion area (data not shown). The shear stress profile plot was taken across the chamber floor, 12.5 mm from the inlet, and the plot indicates that shear stress varies less than 5% across the width of the channel (data not shown).

C. Cell Adhesion Area

The cell adhesion area includes collagen IV protein adsorbed to a self-assembled monolayer (SAM) of hexadecanethiol molecules. Briefly, the surface of a 2.5 mm-diameter, round polydimethylsiloxane (PDMS) stamp was painted with a 1 mM solution of hexadecanethiol (HDT in 200 proof ethanol). The solution was stamped onto the topographical surface (substrate) and firmly held for 30 seconds. After 30 seconds, the stamp was released. The surface was then back-flooded with 2 mM PEG (in 200 proof ethanol) for 2 hours. The PEG solution was removed via aspiration, and the surface was rinsed thoroughly with ethanol. The substrate was then incubated in a 30 $\mu$g/ml solution of collagen IV (in PBS) for 3 hours at room temperature. The collagen solution was removed by aspiration, and the substrate was rinsed thoroughly with PBS.

The SAM provides one exemplary model chemistry which provides consistent and characterizable adsorption of the extracellular matrix (ECM) protein, resulting in a repeatable ECM-based cue to the cells adhered. The SAM also enables chemical patterning of the surface through micro-contact printing ($\mu$CP), which allows selective placement of cells in applications such as multiple phenotype co-culture and cell morphology influence. The collagen IV coating covers both the tops of ridges as well as surfaces within the grooves, as examined by SEM (data not shown). The combination of topographic patterning and $\mu$CP allows distinct control of both physical and chemical patterns to cue cells according to user-specified configurations.

Human renal proximal tubular epithelial cells (HK-2 cells; ATCC CRL-2190) were seeded on the functionalized ridge/groove surface of the apparatus and grown to confluence as follows. A cell suspension solution (containing media and HK-2s) was placed in a petri dish containing the topographically- and chemically-patterned substrate so that the initial seeding density was 300 cells/mm$^2$. Media was DMEM/F12 base supplemented with 0.5% FBS, 10 ng/mL hEGF, 5 $\mu$g/mL insulin, 0.5 $\mu$g/mL hydrocortisone, 0.5 $\mu$g/mL epinephrine, 6.5 ng/mL tri-iodothyronine, 10 $\mu$g/mL transferrin, 100 U/ml penicillin and 100 $\mu$g/mL streptomycin. Cells were placed in an incubator at 37°C and 5% CO$_2$. Media was renewed every other day. Cells reached confluency within 3-4 days. Cells were then exposed to 2 hours of either 0, 0.02 or 1.0 dyn/cm2 FSS under the same conditions as described above.

D. Results

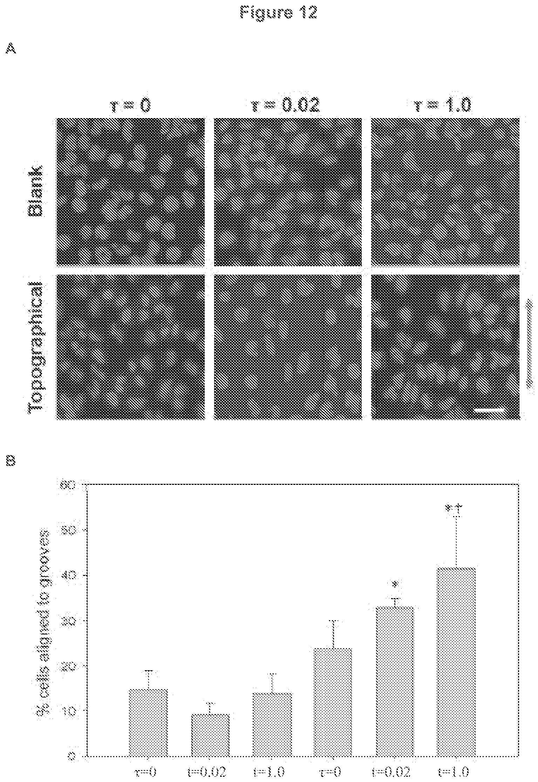

Results are shown in Figure 12. Cells on textured substrates exhibited alignment to grooves, while cells on blank substrates did not (Figure 12 A; the arrow indicates the direction of grooves on topographical substrates). The percentage of nuclei aligned to grooves within 10° increased significantly due to presence of grooves and FSS (Figure 12 B). Regarding Figure 12 B: the first three bars represent cells grown on blank (non-textured) substrate and exposed to 0, 0.02 or 1.0 dyn/cm$^2$ FSS. The last three bars represent cells grown on textured substrate and exposed to 0, 0.02 or 1.0 dyn/cm$^2$ FSS. The presence of 1 dyn/cm$^2$ FSS significantly increased alignment of nuclei for cells adherent to topographic substrates. Substantial improvement was also seen at 0.02 dyn/ cm$^2$ FSS. Data is presented as mean $\pm$ standard deviation. *, P < 0.001 versus blank, $\tau$=0 samples, †, P < 0.005 versus topographical, $\tau$ =0 substrates. (Scale bar, 30 $\mu$m).

Shear stress and topography both influence formation of tight junctions ("TJs") in HK-2 cells. Intensity of TJ formation was measured by quantifying ZO-1 intensities around perimeters of cells on blank and topographical substrates under FSS conditions. ZO-1 was visualized as follows. Cells were permeablilized with 0.5% Triton X-100 in PBS followed by blocking with 1% BSA for 30 minutes at room temperature. Cells were incubated with primary antibodies (ZO-1 594, Invitrogen) for 1 hour at room temperature. Cells were washed 3X in PBS and incubated with secondary antibody (goat anti-mouse IgG, Alexa Fluor 488, Invitrogen) for 1 hour at room temperature. Cells were washed 3X in PBS and coverslips were mounted to slides with ProLong Gold plus DAPI (Invitrogen) and allowed to cure overnight before microscopic analysis. Image analysis included measuring the intensity of the ZO-1 label using a conventional computer imaging program to determine fluorescent intensity of the ZO-1. The intensities were quantified for all borders of cells and the continuity of the junctions was quantified as well.

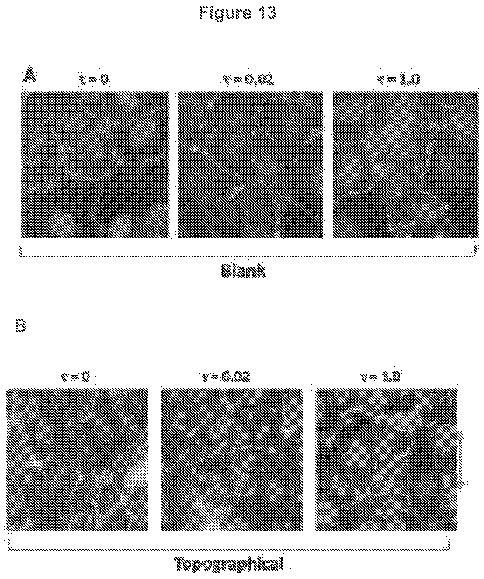

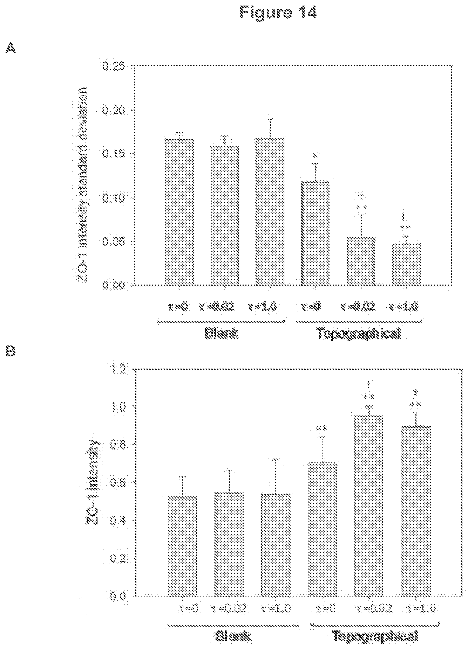

Results are shown in Figures 13 and 14. Figure 13A and B shows representative images of ZO-1 expression for cells cultured on blank and topographical substrates and exposed to either 0, 0.02 or 1.0 dyn/cm2 FSS. With the addition of topography and FSS stimuli, morphology of the ZO-1 borders transitions from punctuate to continuous. The arrow indicates the direction of ridge/groove topography. Figure 14A shows the intensity of ZO-1, integrated along cell perimeters and normalized by cell perimeter, quantified tight junction expression and distribution. The ZO-1 intensity increased significantly in cells cultured on topographical substrates compared to those on blank surfaces. Cells exposed to all levels of FSS on topographical substrates showed a significant increase in ZO-1 intensity compared to cells on topographical substrates exposed to $\tau = 0$ conditions. Figure 14 C shows the standard deviation of ZO-1 intensity measured along cell perimeters and quantifies tight junction continuity. Standard deviation of ZO-1 intensity decreased for all topographical samples compared to cells on blank surfaces and was lowest for cell populations exposed to both topographical substrates and FSS. Cell populations on blank surfaces did not present ZO-1 intensity differences after two hours of FSS. Data is presented as mean $\pm$ standard deviation. *, P < 0.05 versus blank, $\tau = 0$ samples; **, P < 0.001 versus blank, $\tau = 0$ samples; †, P < 0.001 versus topographical $\tau = 0$ samples. (Scale bar, 15 $\mu$m).

The perimeters of cells, as defined by the ZO-1 TJs, transition to a higher intensity with more fluorescence signal as topography and FSS are applied. Overall fluorescent signal, as well as location of the fluorescent signal, indicates increased ZO-1 expression and translocation to cell perimeters. Standard deviation of the ZO-1 intensity around the cell perimeter serves as a quantifiable metric of TJ continuity. Lower standard deviations translate to a more continuous ZO-1 perimeter. Cell perimeters transition from a more punctuate morphology to a continuous morphology with the application of topography and FSS, as measured by ZO-1 intensity standard deviation. Taken together, increases in TJ intensity and continuity indicate a progression towards a quality barrier function for cells grown with topographical substrate and FSS exposure, and a move towards tubule-specific function. The enhancement of cell response to FSS on topographical substrates indicates the synergistic influence of these two physical stimuli. Cells cued by the biomimetic flow apparatus which exhibit high and continuous intensity of ZO-1- labeled TJs are likely poised to form a well-developed, highly functioning epithelial layer with the natural filtering behavior of the renal proximal tubule.

## Claims

1. An apparatus comprising:

    a microfluidic flow channel having at least one surface with ridges and grooves parallel to the direction of flow through the flow channel and having-two different topographical patterns formed therein, the two different topographical patterns being distinct regions having different groove widths, the two topographical patterns of the surface are of micron-scale, the two different topographical patterns used to mimic an in vivo structure having two distinct cell types or arrangements; and

    an inlet in fluid connection with the flow channel for allowing fluid to flow into the flow channel.

2. The apparatus of claim 1, wherein the two different topographical patterns of the surface are selected to promote increased adhesion of cells in the cell layer to the at least one surface.

3. The apparatus of claim 1, further comprising a fluid source for flowing a fluid through the flow channel via the inlet, wherein the fluid induces a shear stress upon the cell layer.

4. The apparatus of claim 3, wherein the flow channel can be disassembled for examining the cell layer after flowing the fluid through the flow channel; or wherein the fluid source is configured to flow the fluid at a flow rate that results in a level of shear stress on the cell layer that is less than $10^{-6}$ N/cm$^2$ (0.1 dyn/cm$^2$).

5. The apparatus of claim 1, wherein the two different topographical patterns of the surface comprise ridges having a width of about 5 micron or less.

6. The apparatus of claim 1, wherein the two different topographical patterns of the surface comprise grooves having a widths in the range of 20 nm to 5 microns.

7. The apparatus of claim 1, comprising a cytophilic substance disposed on a portion of a substrate for growing the cell layer in the portion of the substrate, and wherein the portion of the substrate forms a surface of the flow channel.

8. The apparatus of claim 1, comprising a cytophobic substance disposed on a portion of a surface of a substrate for preventing growth of the cell layer in the portion of the substrate, and wherein the portion of the substrate does, or does not, form a surface of any flow channel.

9. The apparatus of claim 1, wherein the two different topographical patterns of the surface cause the arrangement, behavior, or morphology of the cell layer to replicate an arrangement, behavior, or morphology of cells in a kidney.

10. The apparatus of claim 1, further comprising at least a second flow channel, wherein at least one surface of the second flow channel has a topography formed therein.

11. A method for simulating organ performance comprising:

providing a flow apparatus comprising:

a microfluidic flow channel having at least one surface with ridges and grooves parallel to a direction of the flow channel and having two different topographical patterns formed therein, the two different topographical patterns being distinct regions having different groove widths, , the two topographical patterns of the surface are of micron-scale, the two different topographical patterns used to mimic an *in vivo* structure having two distinct cell types or arrangements; and
a cell layer, having the two distinct cell types or arrangements over the two different topographical patterns

selecting a flow rate for a fluid that, when flowed through the flow channel, results in a level of shear stress on the cell layer that causes cells in the cell layer to mimic the morphology, behavior, or arrangement they would exhibit *in vivo*; and
introducing a fluid flow through the flow channel at the selected flow rate.

12. The method of claim 11, wherein the flow channel is shaped such that the fluid flow results in a plurality of shear stress levels along a length of the flow channel, the method further comprising:

selecting a flow rate that results in a plurality of levels of shear stress on the cell layer that mimics shear stress levels that the cells in the cell layer would experience in different positions of the organ *in vivo*.

13. The method of claim 11, wherein the fluid contains a therapeutic agent, and the method further comprising analyzing the cells to determine the efficacy of the therapeutic agent on the cell layer.

**Patentansprüche**

1. Vorrichtung, die Folgendes umfasst:

einen mikrofluidischen Strömungskanal, der mindestens eine Oberfläche mit Rippen und Rillen parallel zu der Richtung der Strömung durch den Strömungskanal aufweist und zwei verschiedene topografische Muster darin ausgebildet aufweist, wobei die zwei verschiedenen topografischen Muster unterschiedliche Bereiche sind, die verschiedene Rillenbreiten aufweisen, die zwei topografischen Muster der Oberfläche im Mikrometerbereich liegen, die zwei verschiedenen topografischen Muster, die zur Nachahmung einer in-vivo-Struktur verwendet werden, zwei unterschiedliche Zelltypen oder -anordnungen aufweisen; und

einen Einlass in Flüssigkeitsverbindung mit dem Strömungskanal, um zu ermöglichen, dass die Flüssigkeit in den Strömungskanal fließt.

2. Vorrichtung nach Anspruch 1, wobei die zwei verschiedenen topografischen Muster der Oberfläche ausgewählt sind, um erhöhte Adhäsion von Zellen in der Zellschicht an die mindestens eine Oberfläche zu fördern.

3. Vorrichtung nach Anspruch 1, die weiter eine Flüssigkeitsquelle für das Fließen einer Flüssigkeit durch den Strömungskanal über den Einlass umfasst, wobei die Flüssigkeit eine Scherspannung auf der Zellschicht auslöst.

4. Vorrichtung nach Anspruch 3, wobei der Strömungskanal zur Untersuchung der Zellschicht nach dem Fließen der Flüssigkeit durch den Strömungskanal zerlegt werden kann; oder wobei die Flüssigkeitsquelle konfiguriert ist, dass die Flüssigkeit in einer Flussrate fließt, die zu einer Höhe an Scherspannung auf der Zellschicht führt, die weniger als $10^{-6}$ N/cm$^2$ (0,1 dyn/cm$^2$) beträgt.

5. Vorrichtung nach Anspruch 1, wobei die zwei verschiedenen topografischen Muster der Oberfläche Rippen umfassen, die eine Breite von etwa 5 Mikron oder weniger aufweisen.

6. Vorrichtung nach Anspruch 1, wobei die zwei verschiedenen topografischen Muster der Oberfläche Rillen umfassen, die Breiten im Bereich von 20 nm bis 5 Mikron aufweisen.

7. Vorrichtung nach Anspruch 1, die eine zytophile Substanz umfasst, die auf einem Teil eines Substrats zum Wachsen der Zellschicht in dem Teil des Substrats angeordnet ist, und wobei das Teil des Substrats eine Oberfläche des Strömungskanals bildet.

8. Vorrichtung nach Anspruch 1, die eine zytophobe Substanz umfasst, die auf einem Teil einer Oberfläche eines Substrats zur Verhinderung von Wachstum der Zellschicht in dem Teil des Substrats angeordnet ist, und wobei das Teil des Substrats eine Oberfläche eines jedweden Strömungskanals bildet oder nicht bildet.

9. Vorrichtung nach Anspruch 1, wobei die zwei verschiedenen topografischen Muster der Oberfläche bewirken, dass die Anordnung, das Verhalten oder die Morphologie der Zellschicht eine Anordnung, ein Verhalten oder eine Morphologie von Zellen in einer Niere reproduzieren.

10. Vorrichtung nach Anspruch 1, die weiter mindestens einen zweiten Strömungskanal umfasst, wobei mindestens eine Oberfläche des zweiten Strömungskanals eine Topografie darin ausgebildet aufweist.

11. Verfahren zur Simulierung von Organleistung, das Folgendes umfasst:

Bereitstellen einer Strömungsvorrichtung, die Folgendes umfasst:

einen mikrofluidischen Strömungskanal, der mindestens eine Oberfläche mit Rippen und Rillen parallel zu einer Richtung des Strömungskanals aufweist und zwei verschiedene topografische Muster darin ausgebildet aufweist, wobei die zwei verschiedenen topografischen Muster unterschiedliche Bereiche sind, die verschiedene Rillenbreiten aufweisen, die zwei topografischen Muster der Oberfläche im Mikrometerbereich liegen, die zwei verschiedenen topografischen Muster, die zur Nachahmung einer *in-vivo*-Struktur verwendet werden, zwei unterschiedliche Zelltypen oder -anordnungen aufweisen;
und
eine Zellschicht, die zwei unterschiedliche Zelltypen oder -anordnungen über den zwei verschiedenen topografischen Mustern aufweist;

Auswählen einer Flussrate für eine Flüssigkeit, die, wenn sie durch den Strömungskanal fließt, zu einer Höhe an Scherspannung auf der Zellschicht führt, die bewirkt, dass die Zellen in der Zellschicht die Morphologie, das Verhalten oder die Anordnung nachahmen, die sie *in vivo* zeigen würden; und
Einführen einer Flüssigkeitsströmung durch den Strömungskanal bei der ausgewählten Flussrate.

12. Verfahren nach Anspruch 11, wobei der Strömungskanal derart geformt ist, dass die Flüssigkeitsströmung zu einer Vielzahl von Scherspannungshöhen entlang einer Länge des Strömungskanals führt, wobei das Verfahren weiter Folgendes umfasst:

Auswählen einer Flussrate, die zu einer Vielzahl von Höhen an Scherspannung auf der Zellschicht führt, die Scherspannungshöhen nachahmt, die die Zellen in der Zellschicht in verschiedenen Positionen des Organs *in vivo* erfahren würden.

13. Verfahren nach Anspruch 11, wobei die Flüssigkeit ein Therapeutikum enthält und das Verfahren weiter das Analysieren der Zellen umfasst, um die Wirksamkeit des Therapeutikums auf die Zellschicht zu bestimmen.

## Revendications

1. Appareil comprenant :

un canal d'écoulement microfluidique ayant au moins une surface avec des nervures et des rainures parallèles à la direction de l'écoulement à travers le canal d'écoulement et ayant deux motifs topographiques différents formés dans celle-ci, les deux motifs topographiques différents étant des régions distinctes ayant des largeurs de rainures différentes, les deux motifs topographiques de la surface sont à l'échelle du micron, les deux motifs topographiques différents utilisés pour imiter une structure in vivo ayant deux types ou agencements de cellules distincts ; et

une admission en connexion fluidique avec le canal d'écoulement pour permettre l'écoulement de fluide jusque dans le canal d'écoulement.

2. Appareil selon la revendication 1, dans lequel les deux motifs topographiques différents de la surface sont sélectionnés pour promouvoir l'augmentation de l'adhérence des cellules dans la couche de cellules au au moins une surface.

3. Appareil selon la revendication 1, comprenant en outre une source de fluide pour amener un fluide à s'écouler à travers le canal d'écoulement par l'admission, dans lequel le fluide induit une contrainte de cisaillement sur la couche de cellules.

4. Appareil selon la revendication 3, dans lequel le canal d'écoulement peut être désassemblé pour examiner la couche de cellules après avoir amené le fluide à s'écouler à travers le canal d'écoulement ; ou dans lequel la source de fluide est configurée pour amener le fluide à s'écouler à un débit qui a pour résultat un niveau de contrainte de cisaillement sur la couche de cellules qui est inférieur à $10^{-6}$ N/cm$^2$ (0,1 dyn/cm$^2$).

5. Appareil selon la revendication 1, dans lequel les deux motifs topographiques différents de la surface comprennent des nervures ayant une largeur d'environ 5 microns ou moins.

6. Appareil selon la revendication 1, dans lequel les deux motifs topographiques différents de la surface comprennent des rainures ayant des largeurs dans la plage de 20 nm à 5 microns.

7. Appareil selon la revendication 1, comprenant une substance cytophile disposée sur une partie d'un substrat pour cultiver la couche de cellules dans la partie du substrat, et dans lequel la partie du substrat forme une surface du canal d'écoulement.

8. Appareil selon la revendication 1, comprenant une substance cytophobe disposée sur une partie d'une surface d'un substrat pour prévenir la croissance de la couche de cellules dans la partie du substrat, et dans lequel la partie du substrat forme, ou non, une surface d'un quelconque canal d'écoulement.

9. Appareil selon la revendication 1, dans lequel les deux motifs topographiques différents de la surface font que l'agencement, le comportement, ou la morphologie de la couche de cellules reproduisent un agencement, un comportement ou une morphologie de cellules dans un rein.

10. Appareil selon la revendication 1, comprenant en outre au moins un deuxième canal d'écoulement, dans lequel au moins une surface du deuxième canal d'écoulement a une topographie formée dans celle-ci.

11. Procédé pour simuler la performance d'un organe comprenant :

la fourniture d'un appareil d'écoulement comprenant :

un canal d'écoulement microfluidique ayant au moins une surface avec des nervures et des rainures parallèles à une direction du canal d'écoulement et ayant deux motifs topographiques différents formés dans celle-ci, les deux motifs topographiques différents étant des régions distinctes ayant des largeurs de rainures différentes, les deux motifs topographiques de la surface sont à l'échelle du micron, les deux motifs topographiques différents utilisés pour imiter une structure in vivo ayant deux types ou agencements de cellules distincts ; et

une couche de cellules, ayant les deux types ou agencements de cellules distincts au-dessus des deux motifs topographiques différents

la sélection d'un débit pour un fluide qui, quand il est amené à s'écouler à travers le canal d'écoulement, a pour résultat un niveau de contrainte de cisaillement sur la couche de cellules qui fait que les cellules dans la couche de cellules imitent la morphologie, le comportement ou l'agencement qu'elles révéleraient in vivo ; et l'introduction d'un écoulement de fluide à travers le canal d'écoulement au débit sélectionné.

12. Procédé selon la revendication 11, dans lequel le canal d'écoulement est façonné de sorte que l'écoulement de fluide a pour résultat une pluralité de niveaux de contrainte de cisaillement le long d'une longueur du canal d'écoulement, le procédé comprenant en outre :

la sélection d'un débit qui a pour résultat une pluralité de niveaux de contrainte de cisaillement sur la couche de cellules qui imite les niveaux de contrainte de cisaillement que les cellules dans la couche de cellules connaîtraient dans des positions différentes de l'organe in vivo.

13. Procédé selon la revendication 11, dans lequel le fluide contient un agent thérapeutique, et le procédé comprend en outre l'analyse des cellules pour dé-

**EP 2 721 141 B1**

terminer l'efficacité de l'agent thérapeutique sur la couche de cellules.

Figure 1A

Figure 1B

Figure 1C

Figure 1D

Figure 2A

Figure 2B

240

Mounting Apparatus
242

Assembled Flow
Apparatus
200

Observational
Equipment
244

—directed at—

regulates
CO₂ level of

Carbon Dioxide
Controller
270

injects
fluid
into

detects
temperature of

—heats—

Fluid Injection System

Syringe Pump
254

—controls—

Syringe
252

250

Temperature Control System

Temperature
Sensor
262

Heating
Element
266

sends
temperature to

sends
commands to

Temperature
Controller
264

260

Figure 2C

EP 2 721 141 B1

<u>300</u>

```
┌─────────────────┐
│ Obtain surface  │ ──── 302
│   having a      │
│   topography    │
└─────────────────┘
        │
        ▼
┌─────────────────┐
│Chemically pattern│ ──── 304
│   topographic   │
│     surface     │
└─────────────────┘
        │
        ▼
┌─────────────────┐
│ Grow cell culture│ ──── 306
│     layer       │
└─────────────────┘
        │
        ▼
┌─────────────────┐
│ Assemble flow   │ ──── 308
│    channel      │
└─────────────────┘
        │
        ▼
┌─────────────────┐
│ Flow fluid through│ ──── 310
│  flow channel   │
└─────────────────┘
        │
        ▼
┌─────────────────┐
│  Fix cells for  │ ──── 312
│    analysis     │
└─────────────────┘
```

Figure 3

Figure 4

500

```
┌─────────────────────────┐
│ Paint PDMS stamp with   │────── 502
│ hydrophobic solution    │
└─────────────────────────┘
            │
            ▼
┌─────────────────────────┐
│ Stamp solution onto     │────── 504
│ topographical surface   │
└─────────────────────────┘
            │
            ▼
┌─────────────────────────┐
│ Back-flood with cytophobic │──── 506
│ solution                │
└─────────────────────────┘
            │
            ▼
┌─────────────────────────┐
│ Coat topographical surface │──── 508
│ with ECM protein        │
└─────────────────────────┘
            │
            ▼
┌─────────────────────────┐
│ Place topographical surface │── 510
│ in Petri dish           │
└─────────────────────────┘
            │
            ▼
┌─────────────────────────┐
│ Fill Petri dish with cell │──── 512
│ suspension              │
└─────────────────────────┘
            │
            ▼
┌─────────────────────────┐
│ Remove growth media from │──── 514
│ the Petri dish          │
└─────────────────────────┘
            │
            ▼
┌─────────────────────────┐
│ Assemble microchannel on │──── 516
│ top of topographical surface │
└─────────────────────────┘
```

Figure 5

Figure 6A

Figure 6B

Figure 6C

Figure 6D

Figure 7A

Figure 7B

Figure 7C

direction
of flow

Figure 7D

Figure 8A

Figure 8B

Figure 8C

Figure 8D

Figure 8E

Figure 8F

```
┌─────────────────────┐
│ Flow fluid containing│ ─── 902
│potential toxin through│
│    flow channel     │
└─────────────────────┘
          │
          ▼
┌─────────────────────┐
│                     │ ─── 904
│  Fix cells to preserve│
│                     │
└─────────────────────┘
          │
          ▼
┌─────────────────────┐
│  Stain cells to enhance│ ─── 906
│      contrast       │
└─────────────────────┘
          │
          ▼
┌─────────────────────┐
│   Examine cells to  │ ─── 908
│   analyze toxicity  │
└─────────────────────┘
```

Figure 9A

```
┌─────────────────────┐
│ Flow fluid containing│ ─── 952
│  therapeutic agent  │
│  through flow channel│
└─────────────────────┘
          │
          ▼
┌─────────────────────┐
│                     │ ─── 954
│  Fix cells to preserve│
│                     │
└─────────────────────┘
          │
          ▼
┌─────────────────────┐
│  Stain cells to enhance│ ─── 956
│      contrast       │
└─────────────────────┘
          │
          ▼
┌─────────────────────┐
│   Examine cells to  │ ─── 958
│  analyze therapeutic│
│       efficacy      │
└─────────────────────┘
```

Figure 9B

EP 2 721 141 B1

**A**

**B**

**C**

**E** Outside channel wall

Confluent layer of cells

**D**

PDMS channel

Renal tubule cells

# Figure 10

EP 2 721 141 B1

Figure 11

Figure 12

A

B

Figure 13

Figure 14

A

B

**REFERENCES CITED IN THE DESCRIPTION**

**Patent documents cited in the description**

- WO 2006037033 A

- WO 2010124227 A